# EUROPEAN PATENT APPLICATION

(11) **EP 1 618 797 A1**
(43) Date of publication of application: **25.01.2006**
(21) Application number: 04254136.7
(22) Date of filing: 09.07.2004
(51) Int. Cl.: A23L 1/22, A23G 3/00, A61K 9/00, A23L 1/236, A23L 1/054

(54) **Method of producing edible films including aspartame**

(71) Applicant: WM. WRIGLEY JR. COMPANY, Chicago, Illinois 60611-4287 (US)
(72) Inventor: Barabolak, Roman M., Palos Park, IL 60464-2548 (US); Chapdelaine, Albert H., Naperville Illinois (US); Hook, Jeffrey S., Lockport, IL 60441-4386 (US); Zyck, Daniel J., North Riverside Illinois (US); Grey, Ronald T., Morton Grove Illinois (US); Levakov, Vukasin, Chicago IL 60640-3949 (US)
(74) Representative: Hayes, Adrian Chetwynd

(57) **Abstract**

A method of producing edible thin film products comprising the steps of:
adding to a film formulation a food grade acid; and
adding aspartame to the film formulation and acid.

## Description

### BACKGROUND OF THE INVENTION

The present invention generally relates to edible film compositions. More specifically, the present invention relates to edible films with increased stability.

Aspartame has been used as an artificial, low calorie sweetener in numerous food, cosmetic, and pharmaceutical compositions. Aspartame is a very attractive sweetener due to its intensity and because very low levels can be used to achieve a high sweetness perception by the consumer. It is noted for its clean, sweet taste that resembles sucrose. Aspartame has recently become a desirable sweetener to employ in edible film products.

Edible film products are designed to adhere to and rapidly dissolve in the mouth of the consumer. Edible films can provide flavor and/or oral care agents, e.g., breath freshening to the consumer. Such films typically include a film former and flavor or other ingredient. See, for example, U.S. Patent No. 5,948,430 and U.S. Application Publication No. US2001/0022964A1.

Edible film products are usually provided to the consumer in strip form. The strips are sized so that they can be placed on the tongue of a consumer. In this regard, the edible film strips typically have a size of a postage stamp or slightly larger. These strips preferably have a supple texture and are non-self adhering.

One type of edible film product is distributed by Pfizer Consumer Healthcare under the name Listerine® PocketPaks™. The Pfizer edible film product is packaged in a plastic container that includes a top that can open along a lunge. A stack of strips are located in an interior of the package one on top of another. The package is designed so that the consumer can open the container and remove one strip from the stack with his or her finger.

Edible film products are also disclosed in Assignee's co-pending U.S. parent application "Rolled Edible Thin Film Products and Methods of Making Same," filed on August 27, 2002, bearing U.S. Serial No. 10/228,742.

Attempting to incorporate aspartame in an edible film results in a less than satisfactory product. The aspartame can become unstable, yielding an inferior, less flavorful product. The instability of aspartame is primarily due to the nature of aspartame itself; its crystalline structure and configuration. Aspartame has historically exhibited difficulty in maintaining its structure in complex, heterogeneous environments. In particular, aspartame has two major disadvantages. First, aspartame becomes unstable under acidic conditions, and exhibits rapid degradation when exposed to elevated temperatures. Secondly, the peptide nature of aspartame makes it susceptible to hydrolysis; this feature also causes other reactions and microbial degradation.

Aspartame has previously demonstrated instability in other products, such as chewing gums. Sugarless chewing gums often contain flavors and hygroscopic components that are usually saturated with aldehydes that contribute to the instability of aspartame. The components of a chewing gum matrix generally yield a product having a pH of about 4.5. At this pH, aspartame becomes unstable.

Similarly, the production of edible films also provide a complex matrix of ingredients and processing parameters that aid in rendering aspartame unstable. Therefore, there is a need for an edible film product including aspartame that is stable.

### SUMMARY OF THE INVENTION

The present invention provides an edible film product with increased aspartame stability due to the incorporation of acid into the film formulation during processing.

It has been surprisingly discovered That by incorporating acids into the film coating syrup during processing and maintaining a pH range of about 4.5 to about 6.0, the aspartame will not denature in the final film composition, despite the high processing temperatures to which the film is exposed.

In accordance with the present invention, a variety of methods of forming a thin edible film can be used. One method includes the steps of: adding film-forming materials together with water and agitating same until the powder is preferably hydrated and few lumps are present. To this mixture, plasticizers, softening agents, colors, sweeteners, cooling agents, flavors and active ingredients can be blended together to form a homogeneous solution.

It should be noted that an acid may be added at anytime before aspartame is included into the mixture. In a preferred embodiment, the acid is incorporated after water has been added to the film forming materials to make the coating syrup. In an embodiment of the process, a coating syrup with acid is then fed into a hopper, and spread onto a moving substrate through a drying tunnel in which it is exposed to high processing temperatures and exits the drying system as a dried, edible film. Upon exiting the drying system, the film may be rolled, cut, stamped, etched, etc., to yield the desired film form.

In an embodiment of the present invention, at least one food grade acid is added to the coating syrup.

In an embodiment, the acid is added to the coating syrup before water is added.

In an embodiment, the acid is added to the coating syrup after water is added.

In an embodiment, the acid is added to mixing tank before other ingredients are added.

In an embodiment of the present invention, the acid is chosen from the group consisting of: phosphoric acid; citric acid; malic acid, acetic, adipic, benzoic, butyric, formic, fumaric, hexanoic, lactic, propionic, succinic, tartaric, carbonic, and sulforic.

In still another embodiment of the present invention, the acid is encapsulated.

In an embodiment, the acid is spray dried.

In an embodiment, the pH of the coating syrup is between 4.7 and 5.4

In an embodiment, the pH of the coating syrup is between 4.5 and 6.0.

In an embodiment of the present invention, the acid maintains the stability of the aspartame in the edible film composition during processing.

In an embodiment, the edible film composition comprises at least one polysaccharide.

In another embodiment, the edible film composition comprises at least one film-forming agent that is a water-soluble non-starch polysaccharide.

In an embodiment of the present invention, the film-forming agents include a polysaccharide and a softener.

In an embodiment of the present invention, the polysaccharide is pullulan.

In another embodiment of the present invention, the film product is a vehicle for delivering active agents to a consumer.

In a further embodiment of the present invention, a method for preparing a rolled film is provided comprising the steps of: forming a mixture of at least one film forming or coating syrup material in powder form with a food grade acid and water; agitating the mixture preferably until the powder is mostly hydrated and few lumps remain; adding to the mixture at least one ingredient selected from the group consisting of: plasticizers, softening agents, colors, sweeteners, cooling agents, flavors and active ingredients; blending the mixture to obtain a homogeneous solution; spreading the solution onto a moving substrate; drying the solution on the substrate to create a flexible film; and winding the film onto a take-up roll.

In an embodiment, a method for preparing a rolled film is provided comprising forming a mixture of at least one film-forming material in powder form and water, agitating the mixture until the powder is mostly hydrated and few lumps remain, adding to the mixture at least one ingredient selected from plasticizers, softening agents, acids, colors, sweeteners, cooling agents, flavors and active ingredients, then adding aspartame, blending the mixture to obtain a homogeneous solution, spreading the solution onto a moving substrate, drying the solution on the substrate to create a flexible film and winding the film onto a take-up roll.

It is an advantage of the present invention to provide improved edible films.

Another advantage of the present invention is to provide a film composition including an acid that maintains the stability of aspartame during processing.

Moreover, an advantage of the present invention is to provide an improved method of manufacturing edible film products.

Still, an advantage of the present invention is to provide an edible film product including aspartame.

Another advantage of the present invention is to enhance the flavor of the edible film.

Additionally, an advantage of the present invention is to provide methods for stabilizing aspartame.

Furthermore, an advantage of the present invention is that it can be implemented by making relatively minor modifications to an otherwise conventional process.

Additional features and advantages of the present invention are described in, and will be apparent in, the detailed description of the presently preferred embodiments.

### DETALIED DESCRIPTION OF THE INVENTION

The present invention provides stable edible film formulations, containing aspartame, through the addition of acid to the film composition. Moreover, the present invention provides methods for manufacturing edible films containing aspartame.

It has been surprisingly found to be advantageous during thin film processing to add acid to the film formulation. Pursuant to the present invention, aspartame containing stable, flavorful edible thin films can be formed by a variety of different processes.

One such process is as follows: (1) an aqueous solution is formed by blending film-forming materials together with water and acid and are agitated until the powdered materials arc preferably mostly hydrated and few lumps are present; (2) to this mixture, plasticizers, softening agents, colors, sweeteners, cooling agents, and active ingredients are blended together to form a homogeneous solution; and (3) this solution is then cast onto a suitable carrier, and dried to form a film. It should be noted that no particular order is placed on incorporating the ingredients into the mixture; however, it is importartt that the acid is included in the solution before the aspartame is added. It should also be noted that a wide variety of film formulations or coating syrups can be used.

As part of the process, preferably, a carrier is used to produce edible films. The carrier material should be impermeable to the film coating, allowing the film coating to disperse evenly onto the carrier and allowing for the case of removal of the film from the carrier. Examples of suitable carriers include plastic or polyester films, polypropylene, polycarbonate, non-siliconized polyethylene terephthalate film, non-siliconized Kraft paper, polyethylene impregnated Kraft paper, metal belts, voltage or corona treated belts, drum dryers, and polytetrafluroethylene-impregnated glass fabric.

A particularly preferred method of casting the film on the carrier is through a slot die extrusion. By use of multiple extruders and specially constructed dies, it is possible to add multiple color stripes to the product. It is also possible to oscillate the die head to produce wavy lines on the product. The resulting films can be laminated to produce various visual effects.

The casting of the solution onto a suitable carrier material can be performed using any conventional coating technique. Examples of coating techniques include spraying, dipping, comma, coaters, knife over plate, roll over roll, reverse roll, slot die extrusion, and various extrusion techniques. Film thickness can be controlled by adjusting the gap on the coating head, or by applying the desired amount of the solution onto the substrate/carrier. No particular limitation is placed on the thickness of the film layer. The thickness of the film may vary, or a multi-layered film product may be processed, depending on the desired speed of dissolution of the edible film while in the oral cavity.

After coating, the film passes through a dryer for moisture reduction. Drying is carried out through a variety of different means, such as high velocity turbulent hot air, conduction from steam heated slide bed, direct heating or casting of film onto a heated drum or belt, hot or cold air impingement, infrared heating, or any other suitable drying equipment that does not adversely affect the components of the film.

Once the film exits the drying system, the dried film is either taken-up along with its substrate or peeled from the carrier to form a wide roll. As the film exits the drying system, it can be exposed to a number of different types of treatments. If desired, the film may be sprinkled with sugar, starch, flavor, color, color enhancers such as glitter, acids, bioadhesives, actives and texturizers such as candy sprinkles to make specialty edible film products that may be desirable to younger consumers.

After the film has been dried and treated as desired, it may be cut, stamped, etched, punched, etc., into its final chosen size and form. For example, the edible film can be a rolled edible film. Rolled edible films and methods of making the same are disclosed in U.S. Patent Application Serial No. 10/228,742, entitled "Rolled Edible Thin Film Products and Methods of Making the Same," filed on August 27,2002, the disclosure which is incorporated herein by reference.

The formulations of the present invention may be used to generate "specialty" films that can be produced at various points during the film making process. For example, those concepts which appeal to younger consumers. Types of specialty film products include, but are not limited to films that are multi-flavoringp multi-layering, multi-coloring, multi-shapes or forms, texturizing, laminating, printing, graphical designs, "tongue-tattoos", oral sensations, varying dissolution profiles, bioadhesive components, within the oral mucosa of a consumer; alone or in combinations thereof. The films of the present invention are also suitable for food applications beyond direct consumption.

Any suitable water-soluble, film-former can be used to produce a rolled edible thin film product. Suitable film-formers include, but arc not limited to, water-soluble non-starch polysaccharides such as modified celluloses, carboxymethylcellulose (CMC), methylcellulose, hydroxypropylmethylcellulose (HPMC), hydrolyzed gums, xanthan gum, guar gum, locust bean gum, tamarind, agar agar, carrageenan, algins, gum arabic, ghatti gum, Karaya gum, tragacanth gum, konjac, arabinogalactan, larch arabinogalactan, beta-glucan, propylene glycol, pullulan, levan, elsinan, pullulan, pectins, curdlan, chitosan; native starches such as corn starch, waxy maize starch, high-amylose corn starch, potato, tapioca, rice and wheat starch; modified starches such as those that have been acid modified, bleached, oxidized, esterified, etherified, crosslinked, and treated enzymatically; starch hydrolyzed products such as maltodextrin; protein such as gelatin, casein, salts of casein, whey, and protein derived from soybeans; polymers such as polyvinyl pyrrolidone, methycrylate copolymer, and carboxyvinyl copolymers alone or in any combination. In an embodiment, the concentration of the film-forming agent constitutes between approximately 5% to about 60% by dry weights, or approximately 20% to about 40% by dry weight of the final film composition. Further, it should be noted that some film forming agents, such as sodium alginate, will have an impact on the pH of the edible film composition.

Further, any suitable food-grade bulk filler can also be added to the film. This can reduce any "slimy" texture as well as provide structure to the film making it more palatable. In an embodiment, the filler can constitute approximately 1% to about 30% by dry weight of the film, or approximately 5% to about 15% by dry weight of the film, The filler can include microcrystalline cellulose, cellulose polymers, such as wood, magnesium and calcium carbonate, ground limestone, silicates, such as magnesium and aluminum silicate, clay, talc, titanium dioxide, mono-calcium phosphate, di-calcimn phosphate, tri-calcium phosphate, other like bulk fillers or combinations thereof.

If it is desired to use reduced levels of film forming agents, softeners can also be employed to ensure the flexibility of the film, thereby reducing brittleness. The softeners, which are also known as plasticizers, may include tallow, hydrogenated tallow, hydrogenated and partially hydrogenated vegetable oils, cocoa butter, sorbitol and other polyols, glycerin, polyethylene glycol, propylene glycol, invert sugars, corn syrup, lecithin, hydrogenated lecithin, mono-, di- and triglycerides, acetylated monoglycerides, fatty acids (e.g. stearic, palmitic, oleic, and linoleic acids), and combinations thereof. In an embodiment, the softener can constitute 0% to about 20% by dry weight of the film, or approximately 2% to about 10% by dry weight of the film.

Another means of controlling the brittleness of the film is to maintain an adequate moisture level in the film. Preferably, moisture levels should range from approximately 5% to about 20% or approximately 10% to about 15% of the final film product.

Pursuant to the present invention, a variety of acids can be used to maintain the stability of the aspartame during processing or to enhance the flavor of the film. These acids include, but, are not limited to malic, fumaric, adipic, succinic, citric, acetic, lactic, pyruvic, butyric, formic, isocitric, ractocitric, shikimic, quinic, oxalic, glyceric, citramalic, glycolic, glucuronic, galacturonic, aspartic, benzoic, lactarimic, cetostearic, allantoic, pyroglutamic, hydrochloric, hexanoic, carbonic, sulfuric, pyrrolidinonecarboxylic, and tartaric. Further, acids may be added to impact the flavor of the product. For example, malic acid is commonly associated with apples, citric acid with lemons, and tartaric acid with grapes. However, mixtures of different acids may be used in combination to create a desired taste or flavor. Further, these acids may be encapsulated to control their rate of release and interaction with the other components of the film matrix.

A variety of other suitable ingredients can be added to the edible film of the present invention. For example, any suitable medicament for oral cleansing, breath freshening or the like can be added to the film formulation. The medicaments can include, for example, pH control agents, tartar caries control, whitening agents, enzymes, breath freshening agents, anti-plaque/anti-gingivitis agents, saliva stimulating agents, pharmaceutical agents, nutraceutical agents, vitamins, mineral, other like medicaments or combinations thereof.

The edible film formulations or coating syrups of the present invention can also include colorants or coloring agents that can be used in any suitable amount to produce a desired color. Further, the rolled films of the present invention, if desired, can include colored stripes and/or other related designs or shapes to produce color contrasts on the edible rolled film. Coloring agents can include, for example, natural food colors and dyes suitable for food, drug, and cosmetic applications. The colorants are typically known as FD&C dyes and lakes.

A variety of flavoring agents can also be added to the edible films. Any suitable amount and type of artificial and/or natural flavoring agents can be used in any sensorially acceptable fashion. For example, the flavor can constitute approximately 0.1% to about 20% by dry weight of the film, preferably approximately 10% to about 15%. The flavoring agent can include, for example, essential oils, synthetic flavors or mixtures including but not limited to oils derived from plants and fruits such as citrus oil, fruit essences, peppermint oil, spearmint oil, other mint oils, clove oils, oil of wintergreen, anise and the like, flavor oils with germ killing properties such as menthol, eucalyptol, thymol, like flavoring agents or combinations thereof.

The flavor can be enhanced and distributed evenly throughout the product by emulsification. Any suitable amount and type of natural and/or synthetic food-grade emulsifier can be used. For example, the emulsifier can include lecithin, food-grade non-ionic emulsifiers, such as fatty acids (C₁₀-C₁₈), mono and diacyl glycerides, ox bile extract, polyglycerol esters, polyethylene sorbitan esters, propylene glycol, sorbitan monopalmitate, sorbitan tristerate, other like emulsifiers or combinations thereof.

The flavors can be emulsified by any suitable emulsification process, such as mechanical processing, vigorous stirring, intense pressure fluctuations that occur in turbulent flow such as homogenization, sonification, colloid milling and the like. Further, flavors may also be encapsulated or spray dried onto the rolled edible film product to enhance flavor properties or to add texture to the film composition.

Any suitable amount of sweetening agents may also be employed for the present invention. Sugar sweeteners generally include saccharide-contaming components including but not limiting to, sucrose, dextrose, maltose, dextrin, invert sugar, fructose, levulose, galactose, corn syrup solids, and the like, alone or in any combination. Sugarless sweeteners include, but are not limited to, sugar alcohols such as sorbitol, mannitol, xylitol, isomalt, hydrogenated starch hydrolysates, maltitol, and the like, alone or in any combination. However, the low weight of the film products of the present invention generally render these low intensity sweeteners ineffective for purposes of sweetening although they may provide functional benefits.

High intensity artificial sweeteners are preferably used, alone or in combination with the above. Preferred sweeteners include, but are not limited to, sucralose, aspartame, N-substituted APM derivatives such as neotame, salts of acesulfame, alitame, saccharin and its salts, cyclamic acid and its salts, glycyrrhizin, dihydrochalones, thaumatin, monellin, and the like, alone or in any combination. In order to provide enhanced or delayed sweetness, or to provide texture to the rolled film product, it may be desirable to encapsulate the sweetener. Such techniques as wet granulation, wax granulation, spray drying, spray chilling, fluid bed coating, coacervation, and fiber extension may be used to achieve the desired characteristics.

Combinations of sugar and/or sugarless sweeteners may be used in the film product. Additionally, a softening agent may also provide additional sweetness such as with aqueous sugar or alditol solutions.

Cooling agents may also be employed in the present invention, cooling agents include, but are not limited to, menthol, WS3, WS23, Utracool, monomenthyl succinate, alone or in any combination. Again, these cooling agents may be encapsulated or spray dried onto the film to enhance a variety of oral sensations.

Depending on the ingredients being used to make the film product, preservatives may also be employed to ensure the safety and quality of the edible thin film. Suitable preservatives include but are not limited sorbic acid, sodium benzoate, potassium sorbate, methyl p-hydroxybenzoate, sodium propionate, and propyl p-hydroxybenzoate alone or in any combination. In addition, suitable antioxidants can be used.

It should be appreciated that any suitable type, number and arrangement of process procedures or steps (e.g. mixing, heating, drying, cooling, addition of ingredients), process parameters (e.g. temperature, pressure, pH, process times) or the like can be utilized to practice the present invention.

By way of example and not limitation, examples of the present invention will now be given.

### Edible Film Preparation Method:

I) Blending:
   a) Powdered materials (such as film forming agents) are blended together using a ribbon blender or equivalent
   b) Flavors or flavor components/enhancers are blended together using mechanical agitation or equivalent.
2) Mixing:
   a) The mixing tank is turned on and set at desired temperature.
   b) To the mixing tank the blended powdered materials are added.
   c) Water is added to the mixing tank, and begin agitation of the mixing tank.
   d) When all water has been added, the desired acid is added.
   e) Once all blended powders and acid have been added to the mixing tank, sweeteners, flavors, colors, etc. are added as the temperature rises. The blend is maintained at an even temperature, about 105 to about. 115°F. The conditions of the mixing room are maintained at about 70 to about 80°F, 40 to about 50% RH.
3) Drying:
   a) Feed solution into a hopper.
   b) Upon entering the drying system, the film solution is coated onto a moving substrate which passes under a coma bar roll to produce a dry thickness of about 48 to about 52 microns.
   c) Adjust the heater temperature to achieve an exit film temperature of about 215 to about 220°F. This should produce a film having a moisture of about 9 to about 11%.
   d) Drying room conditions are about 70 to about 80°F, 40 to about 50% RH.
4) Cutting/Shaping
   a) The film is removed from the substrate upon exiting the drying system and taken up on a separate roll. Once the rolled dried film cools and sets, it is unwound and cut into its desired final form.

Through quality assurance testing, it was discovered that after the edible films had been processed, the loss of aspartame was in a range of about 20 to about 40% in the fmal product after processing. Several tests were run, incorporating acid into the film composition in order to decrease or stop the losses of aspartame in the final film product. The formulas used and the results from the tests run are as follows:

**% Finished Weight**

| **Ingredient** | **Control** | **Comparative Example 1** |
|---|---|---|
| *Alginate* | 32.0 | 32.0 |
| *Maltodextrin* | 17.0 | 17.0 |
| *Carrageenan* | 12.0 | 12.0 |
| *Cellulose* | 10.0 | 10.0 |
| *Glycerin* | 8.5 | 8.5 |
| *Aspartame* | 1.0 | 1.0 |
| *Citric Acid* | -- | 0.15 |
| *Flavor* | 8.0 | 8.0 |
| *Menthol* | 1.5 | 1.5 |
| *Water* | 10.0 | 9.85 |
| *Total* | 100.0 | 100.0 |

### Test 1:

A syrup holding test was conducted to demonstrate aspartame degradation in the holding tank before the solution was processed and dried to form the final film product Syrup was made with and without citric acid and placed in an oven at 125 to about 133°F. Samples of syrup were taken at 0, 2hr, 5hr, 24hr, 48hr, and 76hr. Aspartame levels were tested, and the results are as follows:

| **Time of Sampling (hrs.)** | **Control (% Aspartame Retained)** | **Exemple 1 (% Aspartame Retained)** |
|---|---|---|
| T= 0 | 100% | 100% |
| T= 1 | 92.0% | 100.0% |
| T= 5 | 87.0% | 98.0% |
| T= 24 | 61.0% | 92.0% |
| T= 48 | 44.0% | 81.0% |
| T= 76 | 34.0% | 73% |

The data above demonstrates a significant retention of aspartame while in acidic solution over time. The control sample experienced a 66% loss of aspartame over a 76 hour period of time. In contrast, Example 1 (containing citric acid) experienced only a 27% loss of aspartame over the given period of time.

### Test 2:

Dried syrup (final film product), was also tested for aspartame levels in the finished product. Random samples were taken off the dried film role in the beginning, middle, and end regions of the roll. Aspartame levels were determined from each region, and results are as follows:

| **Sampling region of the roll** | **Control (% Aspartame Retained)** | **Example 1 (% Aspartame Retained)** |
|---|---|---|
| Beginning | 76% | 100% |
| Middle | 66% | 100% |
| End | 40% | 98% |

The data above demonstrates a significant loss of aspartame over time (approximately 5 hours to complete the entire processing run). The control sample experienced a 60% loss of aspartame over the five hour period, whereas Example 1 (containing citric acid) only lost 2% aspartame.

The following examples illustrate some embodiments of the invention. Of course, may others are possible and are easily formulated by a skilled technician once the fundamental concepts of the invention are grasped.

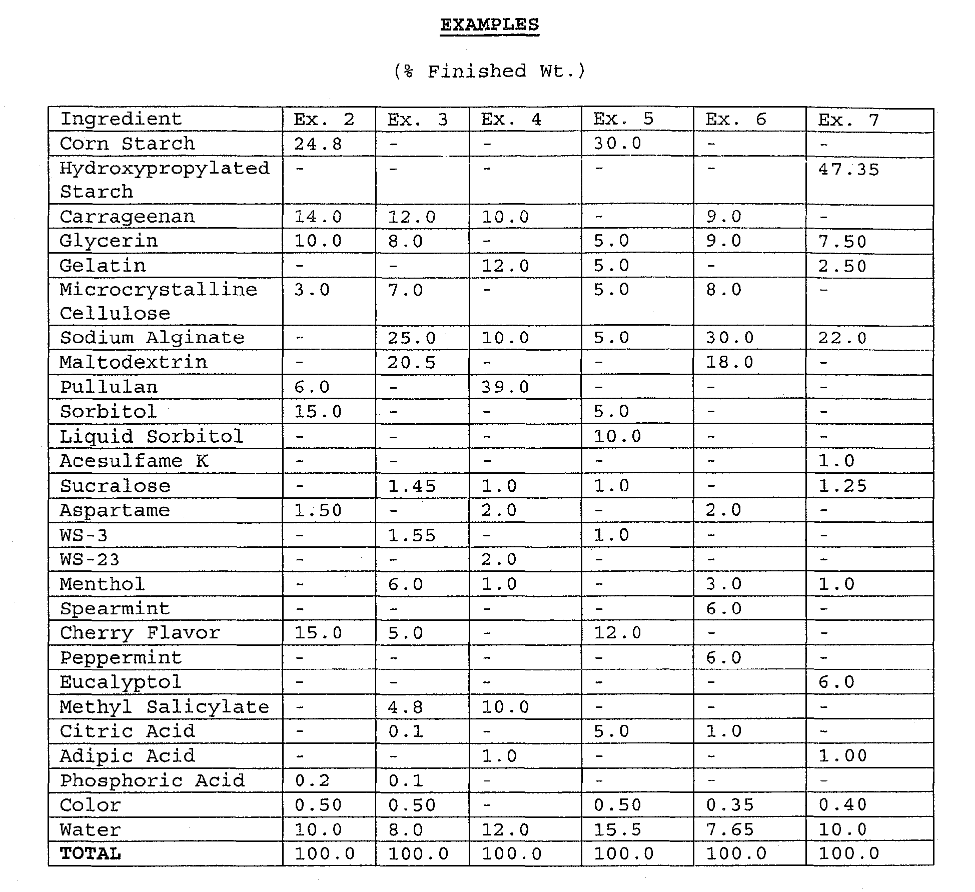

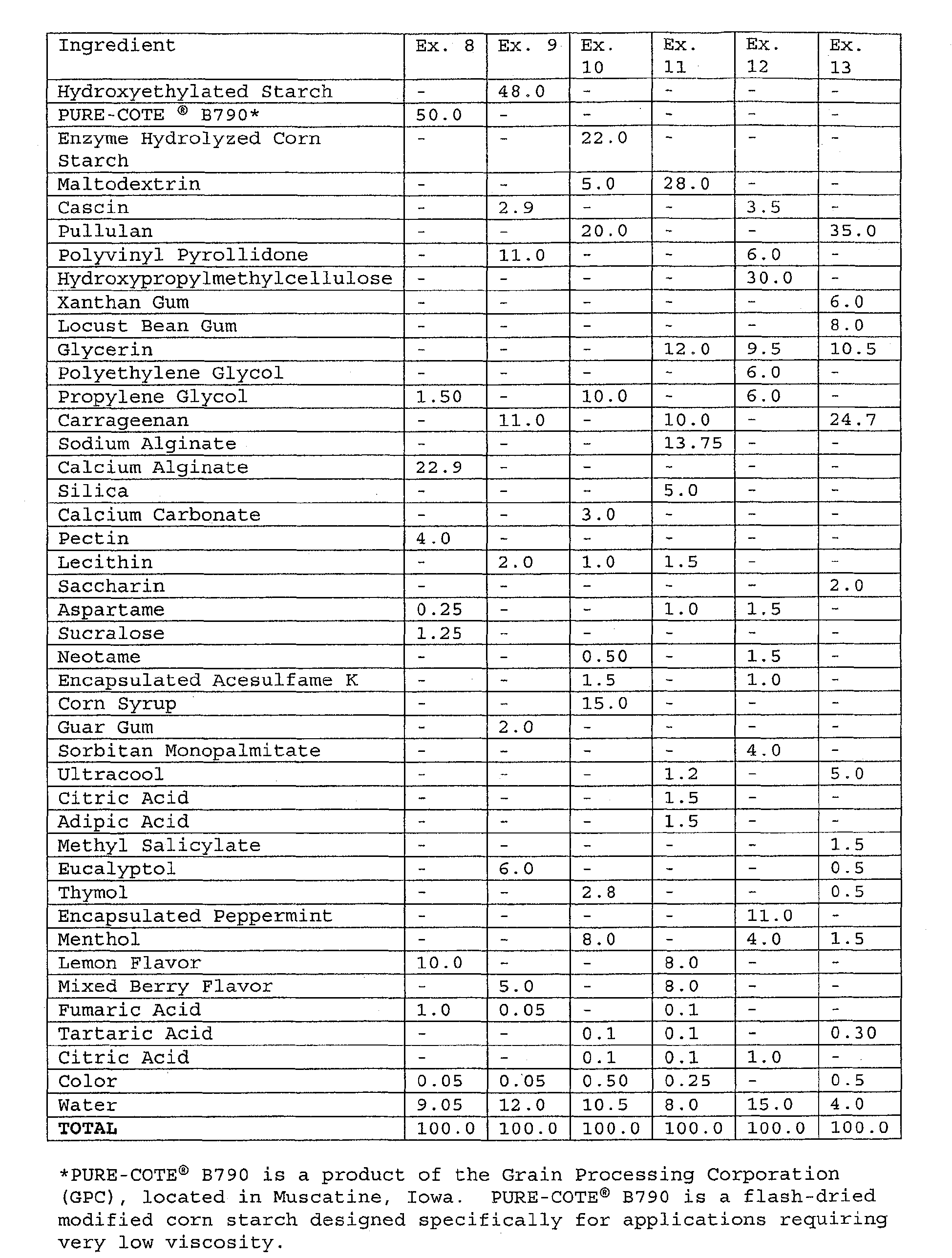

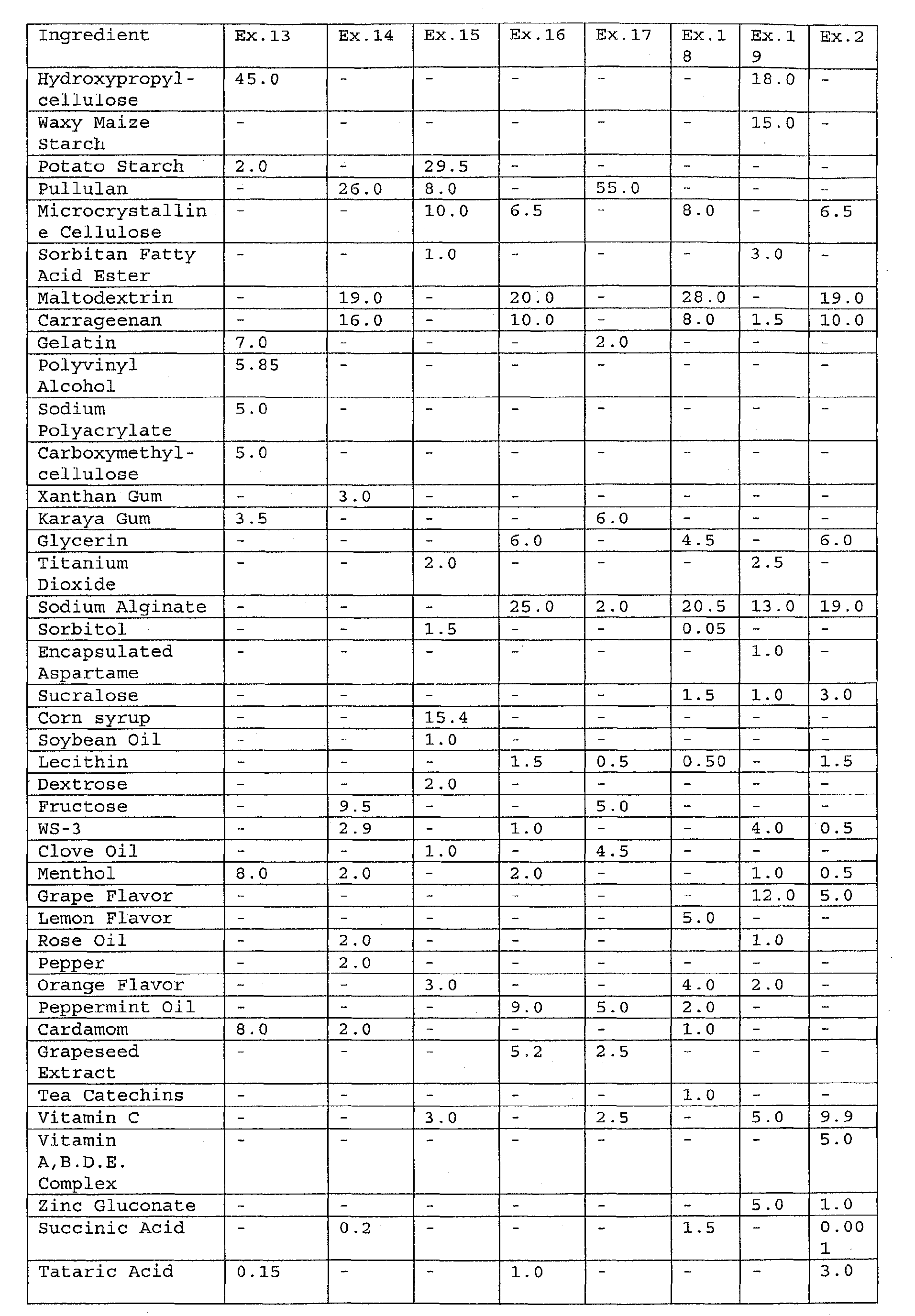

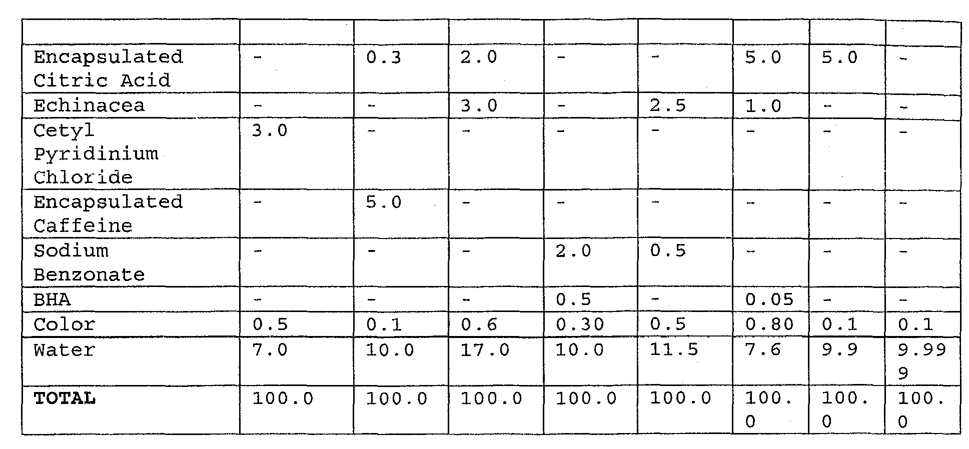

It should be appreciated that various changes and modifications of the presently preferred embodiments described herein will be apparent to those skilled in the art. Such changes and modifications can be made without departing from the original spirit and scope of the present invention without diminishing its intended advantages. It is therefore intended that such changes and modifications be covered by the appended claims.

## Claims

1. A method of producing edible thin film products comprising the steps of:
adding to a film formulation a food grade acid; and
adding aspartame to the film formulation and acid.

2. The method of claim 1 wherein the food grade acid is added to the film formulation before water is added.

3. The method of claim 1 wherein the food grade acid is added to the film formulation after water is added.

4. The method of any one of the preceding claims wherein the film formulation with acid has a pH of 4.5 to 6.0.

5. The method of claim 4 wherein the film formulation with acid has a pH of 4.7 to 5.4.

6. The method of any one of the preceding claims wherein the film formulation includes at least one polysaccharide.

7. The method of any one of the preceding claims wherein the film formulation includes pullulan.

8. A method of producing edible thin films comprising the steps of:
producing a coating syrup that does not include aspartame;
adding to the coating syrup a food grade acid;
adding aspartame to the coating syrup and the food
grade acid; and producing edible film from the coating syrup, aspartame and food grade acid.

9. The method of claim 8 comprising the step of passing the coating syrup through an extruder.

10. A method for preparing a rolled edible film is provided comprising the steps of:
forming a mixture of at least one film-forming material in powder form with a food grade acid and water;
agitating the mixture:
adding to the mixture at least one ingredient selected from the group consisting of plasticizers, softening agents, colors, sweeteners, cooling agents, flavours and active ingredients, blending the mixture to obtain a homogeneous solution;
spreading the solution onto a moving substrate;
drying the solution on the substrate to create a flexible film; and
winding the film onto a take-up roll.

11. The method of claim 10 wherein an extruder is used.

12. The method of any one of claims 8 to 11 wherein the food grade acid is added to the coating syrup before water is added.

13. The method of any one of claims 8 to 11 wherein the food grade acid is added to the coating syrup after water is added.

14. The method of any one of claims 8 to 13 wherein the coating syrup with the food grade acid has a pH of 4.7 to 5.4.

15. The method of any one of claims 8 to 14 wherein the coating syrup includes at least one polysaccharide.

16. The method of any one of claims 8 to 15 wherein the coating syrup includes pullulan.

17. The method of any one of claims 8 to 16 wherein the edible film includes an active agent.

18. The method of any one of claims 8 to 17 wherein the edible film includes a medicament.

19. The method of any one of the preceding claims wherein the food grade acid is chosen from the group consisting of: phosphoric acid, citric acid, malic acid, acetic, adipic, benzoic, butyric, formic, fumaric, hexanoic, lactic, propionic, succinic, tartaric, carbonic, and sulforic.

20. The method of any one of the preceding claims wherein the food grade acid is encapsulated.

21. The method of any one of the preceding claims wherein the food grade acid is spray dried.

22. The method of any one of the preceding claims comprising the step of casting the film formulation and acid using a coating technique.

23. The method of claim 22 wherein the coating technique is chosen from the group consisting of: spraying; dipping; comma cutters; knife over plate; roll over roll; reverse roll and extrusion.

24. The method of claim 22 or 23 comprising a drying step after casting.

25. The method of any one of claims 22 to 24 comprising a roll take up step after casting.
